(19) Europäisches Patentamt
European Patent Office
Office européen des brevets

(11) Numéro de publication: **0 047 206**
**B1**

# (12) FASCICULE DE BREVET EUROPEEN

(45) Date de publication du fascicule du brevet: **23.11.83**

(51) Int. Cl.³: **C 07 C 93/04**

(21) Numéro de dépôt: **81401325.6**

(22) Date de dépôt: **20.08.81**

(54) **Procédé de préparation d'aminoalcools polyoxaalkylés ou polyoxaarylés et leur application comme agents de complexation de cations.**

(30) Priorité: **27.08.80 FR 8018561**

(43) Date de publication de la demande:
**10.03.82 Bulletin 82/10**

(45) Mention de la délivrance du brevet:
**23.11.83 Bulletin 83/47**

(84) Etats contractants désignés:
**AT BE CH DE FR GB IT LI LU NL SE**

(56) Documents cités:
**EP - A - 0 005 094**
**EP - A - 0 016 673**
**FR - A - 2 402 473**
**GB - A - 897 163**

(73) Titulaire: **RHONE-POULENC SPECIALITES CHIMIQUES, "Les Miroirs" 18, Avenue d'Alsace, F-92400 Courbevoie (FR)**

(72) Inventeur: **Linguenheld, Louis, 6, rue d'Illzach, F-68270 Ruelisheim (FR)**
Inventeur: **Soula, Gérard, 33, rue Nungesser, F-69330 Meyzieu (FR)**

(74) Mandataire: **Fabre, Madeleine-France et al, RHONE-POULENC RECHERCHES Service Brevets Chimie et Polymères 25, quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

# Procédé de préparation d'aminoalcools polyoxaalkyles ou polyoxaryles et leur application comme agents de complexation de cations

La présente invention vise un procédé de préparation d'aminoalcools polyoxaalkylés ou polyoxaarylés pouvant être utilisés comme agents de complexation de cations.

Des aminoalcools polyoxaalkylés ont déjà été décrits. Il s'agit notamment (brevet anglais no 897 163) de ceux ayant pour formule:

$$\text{HO-alkylène-N}\left[\text{CH}_2\text{-CH}_2\text{-}(\text{OCH}_2\text{-CH}_2)_{x=2-10}\text{-O-alkyl}_{C_1-C_6}\right]$$

Ils peuvent être préparés par action d'une alcanolamine HO-alkylène-$NH_2$ sur par exemple un chlorure d'alkoxypolyéthylèneoxyéthyl:

$$\text{Cl-CH}_2\text{-CH}_2\text{-}(\text{OCH}_2\text{-CH}_2)_{x=2-10}\text{-O-alkyl}_{C_1-C_6}$$

Il est connu d'après le brevet français no 2 402 473 de préparer des di(hydroxypolyoxaalkyl)mono(alkylétherpolyoxaaryl)amine par oxyéthylation d'une amine à fonction éther obtenue par propoxylation d'un alcool suivie d'une ammonolyse.

Il a également été proposé dans le brevet européen no 5094 de préparer des tris(polyoxaalkyl)amines par action d'un sel de métal alcalin d'un monoalcoyléther d'une éthylène glycol et d'une tris(halogénoéthyl)amine.

Aucun des documents ci-dessus cités ne donne un enseignement quant à la préparation sélective d'aminoalcools présentant un atome d'azote trisubstitué par deux chaînes polyoxaalkylés ou arylés et une chaine polyoxahydroxylée présentant un nombre bien défini de motifs oxyalkylènes.

Selon l'invention le procédé de préparation d'aminoalcools polyoxaalkylés ou polyoxaarylés de formule:

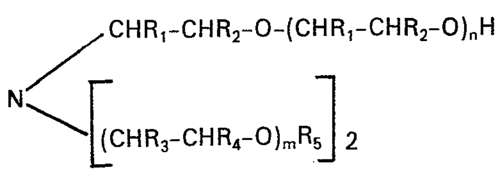

(I)

formule dans laquelle:

- $R_1$, $R_2$, $R_3$ et $R_4$ sont semblables ou différents et représentent:
  - un atome d'hydrogène
  - un radical alkyle contenant de 1 à 4 atomes de carbone
- $R_5$ représente:
  - un radical alkyle contenant de 1 à 12 atomes de carbone, éventuellement substitué par un groupe phényle
  - un radical cycloalkyle contenant de 3 à 12 atomes de carbone
  - un radical phényle éventuellement substitué par un groupe alkyle contenant de 1 à 12 atomes de carbone
- n est un nombre entier supérieur ou égal à 1 et inférieur ou égal à 9
- m est un nombre entier supérieur ou égal à 1 et inférieur ou égal à 10

est caractérisé en ce que l'on fait réagir un polyalkylène glycol de formule:

$$\text{HO-CHR}_1\text{-CHR}_2\text{-O-}(\text{CHR}_1\text{-CHR}_2\text{-O})_n\text{H} \qquad (II)$$

où $R_1$, $R_2$ et n ont la définition donnée ci-dessus avec une bis (polyoxaalkyl)amine ou bis(polyoxaaryl)amine de formule:

$$\text{HN}\left[(\text{CHR}_3\text{-CHR}_4\text{-O})_m\text{R}_5\right]_2 \qquad (III)$$

ou $R_3$, $R_4$, $R_5$ et m ont la définition donnée ci-dessus, selon un rapport molaire polyalkylène glycol de formule (II)/bis(polyoxaalkyl)amine ou bis(polyoxaaryl)amine de formule III d'au moins 1,5 fois le rapport stoechiométriquement requis pour l'obtention d'un aminoalcool polyoxaalkylé ou polyoxaarylé de formule I, en présence d'un catalyseur d'hydrogénation-déshydrogénation, à une température comprise entre 120 et 220°C, de préférence entre 150 et 200°C, et en ce que l'on sépare l'aminoalcool polyoxaalkylé ou polyoxaarylé de formule I.

La présente invention vise à titre préférentiel un procédé de préparation d'aminoalcools polyoxaalkylés ou polyoxaarylés de formule (I) dans lesquels:

- $R_1$, $R_2$, $R_3$, $R_4$ sont semblables ou différents et représentent:
  - un atome d'hydrogène
  - un radical méthyle
- $R_5$ représente:
  - un radical alkyle contenant de 1 à 6 atomes de carbone,

et tout particulièrement de 1 à 4 atomes de carbone,

éventuellement substitué par un groupe phényle;

  - un radical cyclohexyle
  - un radical phényle éventuellement substitué par un groupe alkyle contenant de 1 à 4 atomes de carbone
- n représente un nombre entier supérieur ou égal à 1 et inférieur ou égal à 5, et tout particulièrement égal à 1, 2 ou 3
- m représente un nombre entier supérieur ou égal à 1 et inférieur ou égal à 6, et tout particulièrement égal à 1, 2, 3 ou 4.

A titre d'exemples d'aminoalcools polyoxaalkylés pouvant être préparés selon le procédé de la présente invention, on peut citer:

- le N(hydroxy-5'oxa-3'pentyl)aza-5 dioxa-2,8 nonane

– le N(hydroxy-5′oxa-3′pentyl)aza-6 dioxa-3,9 undecane
– le N(hydroxy-5′oxa-3′pentyl)aza-7 diox-4,10 tridécane
– le N(hydroxy-5′oxa-3′pentyl)aza-8 dioxa-5,11 pentadecane
– le N(hydroxy-5′oxa-3′pentyl)aza-8 tetraoxa-2,5,11,14 pentadecane
– le N(hydroxy-5′oxa-3′pentyl)aza-9 tetraoxa-3,6,12,15 heptadecane
– le N(hydroxy-5′oxa-3′pentyl)aza-10 tetraoxa-4,7,13,16 nonadecane
– le N(hydroxy-5′oxa-3′pentyl)aza-11 tetraoxa-5,8,14,17 heneicosane
– le N(hydroxy-5′oxa-3′pentyl)aza-11 hexaoxa-2,5,8,14,17,20 heneicosane
– le N(hydroxy-5′oxa-3′pentyl)aza-12 hexaoxa-3,6,9,15,18,21 tricosane
– le N(hydroxy-5′oxa-3′pentyl)aza-13 hexaoxa-4,7,10,16,19,22 pentacosane
– le N(hydroxy-5′oxa-3′pentyl)aza-14 hexaoxa-5,8,11,17,20,23 heptacosane

ayant pour formule:

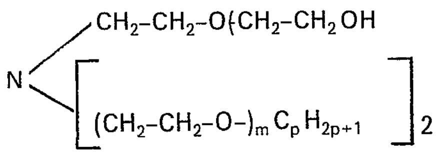

avec m = 1 à 3 et p = 1 à 4

– le N(hydroxy-8′dioxa-3,6′octyl)aza-5 dioxa-2,8 nonane
– le N(hydroxy-8′dioxa-3,6′octyl)aza-6 dioxa-3,9 undecane
– le N(hydroxy-8′dioxa-3,6′octyl)aza-7 dioxa-4,10 tridecane
– le N(hydroxy-8′dioxa-3,6′octyl)aza-8 dioxa-5,11 pentadecane
– le N(hydroxy-8′dioxa-3,6′octyl)aza-8 tetraoxa-2,5,11,14 pentadecane
– le N(hydroxy-8′dioxa-3,6′octyl)aza-9 tetraoxa-3,6,12,15 heptadecane
– le N(hydroxy-8′dioxa-3,6′octyl)aza-10 tetraoxa-4,7,13,16 nonadecane
– le N(hydroxy-8′dioxa-3,6′octyl)aza-11 tetraoxa-5,8,14,17 heneicosane
– le N(hydroxy-8′dioxa-3,6′octyl)aza-11 hexaoxa-2,5,8,14,17,20 heneicosane
– le N(hydroxy-8′dioxa-3,6′octyl)aza-12 hexaoxa-3,6,9,15,18,21 tricosane
– le N(hydroxy-8′dioxa-3,6′octyl)aza-13 hexaoxa-4,7,10,16,19,22 pentacosane
– le N(hydroxy-8′dioxa-3,6′octyl)aza-14 hexaoxa-5,8,11,17,20,23 heptacosane

ayant pour formule:

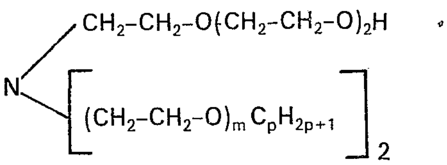

avec m = 1 à 3 et p = 1 à 4

– le N(hydroxy-11′trioxa-3′,6′,9′undecyl)aza-5 dioxa-2,8 nonane
– le N(hydroxy-11′trioxa-3′,6′,9′undecyl)aza-6 dioxa-3,9 undecane
– le N(hydroxy-11′trioxa-3′,6′,9′undecyl)aza-7 dioxa-4,10 tridecane
– le N(hydroxy-11′trioxa-3′,6′,9′undecyl)aza-8 dioxa-5,11 pentadecane
– le N(hydroxy-11′trioxa-3′,6′,9′undecyl)aza-8 tetraoxa-2,5,11,14 pentadecane
– le N(hydroxy-11′trioxa-3′,6′,9′undecyl)aza-9 tetraoxa-3,6,12,15 heptadecane
– le N(hydroxy-11′trioxa-3′,6′,9′undecyl)aza-10 tetraoxa-4,7,13,16 nonadecane
– le N(hydroxy-11′trioxa-3′,6′,9′undecyl)aza-11 tetraoxa-5,8,14,17 heneicosane
– le N(hydroxy-11′trioxa-3′,6′,9′undecyl)aza-11 hexaoxa-2,5,8,14,17,20 heneicosane
– le N(hydroxy-11′trioxa-3′,6′,9′undecyl)aza-12 hexaoxa-3,6,9,15,18,21 tricosane
– le N(hydroxy-11′trioxa-3′,6′,9′undecyl)aza-13 hexaoxa-4,7,10,16,19,22 pentacosane
– le N(hydroxy-11′trioxa-3′,6′,9′undecyl)aza-14 hexaoxa-5,8,11,17,20,23 heptacosane

ayant pour formule:

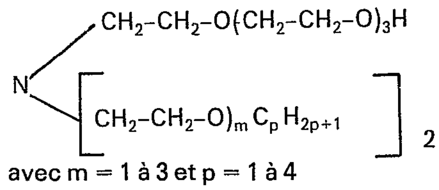

avec m = 1 à 3 et p = 1 à 4

ainsi que les aminoalcools polyoxaarylés ayant pour formule:

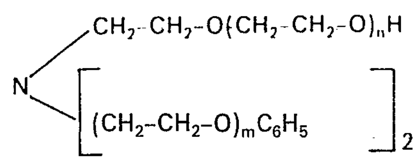

avec n = 1 à 3 et m = 1 à 3

Pour une bonne réalisation du procédé faisant l'objet de la présente invention, on pourra choisir des catalyseurs au nickel, du type nickel Raney ou Harshaw; la quantité de catalyseur pourra être comprise entre 1 et 15% du poids de bis(polyoxaalkyl)amine ou bis(polyoxaaryl)amine et de préférence entre 2 et 6%.

Le rapport molaire polyalkylène glycol de formule (II)/bis (polyoxaalkyl)amine ou bis(polyoxaaryl)amine de formule (III) pourra également être compris entre 1, 5 et 10 fois la stoechiométrie et de préférence entre 2 et 6 fois la stoechiométrie.

Le procédé faisant l'objet de l'invention pourra de préférence être réalisé en présence d'hydrogène, à une pression inférieure à 20 bars, et généralement à pression autogène, sous agitation violente jusqu'à disparition du polyalkylène glycol; cette opération dure de 2 à 10 heures et généralement de 3 à 4 heures. La quantité d'hydrogène pouvant être utilisée est de l'ordre de 1 à 10% du poids d'alkylène glycol mis en œuvre et de préférence de l'ordre de 1 à 5%.

L'eau formée au cours de la réaction pourra être éliminée du milieu réactionnel à l'aide d'un courant gazeux, tel que l'hydrogène quand celui-ci a été mis en œuvre ou de l'azote.

A titre d'exemples de polyalkylène glycol pouvant être mis en œuvre, on peut citer:

- le diéthylène glycol
- le triéthylène glycol
- le tétraéthylène glycol.

A titre d'exemples de bis(polyoxaalkyl)amines ou bis(polyoxaaryl)amines pouvant être mis en œuvre, on peut citer:

- l'aza-5 dioxa-2,8 nonane
- l'aza-6 dioxa-3,9 undecane
- l'aza-7 dioxa-4,10 tridecane
- l'aza-8 dioxa-5,11 pentadecane
- l'aza-8 tetraoxa-2,5,11,14 pentadecane
- l'aza-9 tetraoxa-3,6,12,15 heptadecane
- l'aza-10 tetraoxa-4,7,13,16 nonadecane
- l'aza-11 tetraoxa-5,8,14,17 heneicosane
- l'aza-11 hexaoxa-2,5,8,14,17,20 heneicosane
- l'aza-12 hexaoxa-3,6,9,15,18,21 tricosane
- l'aza-13 hexaoxa-4,7,10,16,19,22 pentacosane
- l'aza-14 hexaoxa-5,8,11,17,20,23 heptacosane

ayant pour formule:

$$HN\left[-(CH_2-CH_2-O)_m C_p H_{2p+1}\right]_2$$

avec m = 1 à 3 et p = 1 à 4,
ainsi que celles ayant pour formule:

$$HN\left[-(CH_2-CH_2O)_m C_6H_5\right]_2$$

avec m = 1 à 3.

Les aminoalcools polyoxaalkylés ou polyoxaarylés obtenus selon le procédé de l'invention peuvent être utilisés comme agents de complexation de cations. La présente invention a également pour objet cette application.

Parmi les cations pouvant être complexés par lesdits aminoalcools polyoxaalkylés ou polyoxaarylés on peut citer: $NH_4^+$ et les cations dérivés des métaux des groupes IA à $VII_A$, VIII, $I_B$ à $V_B$ (Société Chimique de France) en particulier ceux dérivés des métaux alcalins ou alcalino-terreux tels que: Na, K, Li, Cs, Ca, Ba.

La complexation par les aminoalcools polyoxaalkylés ou polyoxaarylés de l'invention, de sels minéraux ou organiques des métaux ci-dessus cités ou de sels d'ammonium, permet de solubiliser lesdits sels ou d'améliorer la solubilité desdits sels dans des solvants organiques dans lesquels ils sont habituellement insolubles ou peu solubles.

Les exemples suivants sont donnés à titre indicatif et ne peuvent être considérés comme une limite du domaine et de l'esprit de l'invention.

Exemple 1

Préparation du N(hydroxy 5′ oxa 3′ pentyl)aza 9 tétraoxa 3,6,12,15 heptadécane:

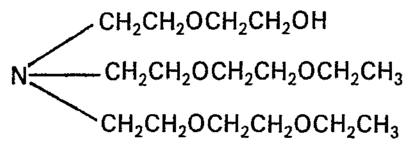

Dans un ballon tétracol de 2 litres, équipé d'une agitation, d'une arrivée d'hydrogène, d'une colonne et d'un condenseur pour recueillir l'eau, on charge:

- diéthylène glycol 1076 g (10,6 moles)
- Ni Raney déshydraté 125 g
- Aza 9 tétraoxa 3,6,12,15 heptadécane 498 g (2 moles)

On agite sous un courant d'hydrogène (1l/mn) et on chauffe 3 heures à 170°C. On recueille 106 g d'eau et de produits légers correspondant à la déshydratation du diéthylène glycol. Après refroidissement, et séparation du Nickel Raney, le mélange est soumis à distillation pour récupérer 888 g de diéthylène glycol et 280 g du produit cherché qui bout à $Eb_1$: 193°.

Le rendement atteint 55% sur l'amine secondaire engagée.

Exemple 2

Préparation du N(hydroxy-8′ dioxa 3′,6′ octyl)aza 9 tétraoxa 3,6,12,15 heptadécane:

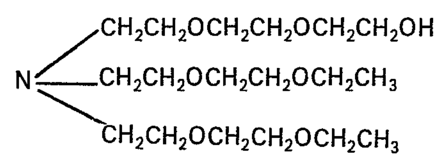

Dans le même appareillage que l'exemple 1, et dans les mêmes conditions de travail, on fait réagir:

- triéthylène glycol 890 g (6 moles)
- Ni Raney déshydraté 160 g
- Aza 9 tétraoxa 3,6,12,15 heptadécane 498 g (2 moles)

Après 3 heures de chauffage à 180°C, puis filtration du Nickel Raney, le mélange est soumis à distillation pour éliminer le triéthylène glycol. On récupère 610 g du produit attendu sous forme d'un liquide brun foncé présentant une pureté de 88,1%.

Le rendement sur l'amine secondaire chargée atteint 71%.

Exemple 3

Préparation du N(hydroxy 8′ dioxa 3′,6′ octyl) aza 11 hexaoxa 2,5,8,14,17,20 heneicosane.

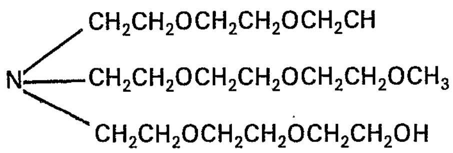

Dans les mêmes conditions qu'à l'exemple 1, on fait réagir:

- aza 11 hexaoxa 2,5,8,14,17,20 heneicosane 250 g (0,80 mole)
- triéthylène glycol 550 g (3,66 moles)
- Nickey Raney déshydraté 50 g

Après 5 heures de réaction à 180° sous courant d'hydrogène, le Nickel Raney est filtré et le filtrat est étété jusqu'à 300° sous 0,1 mmHg (13,3 Pa).

On obtient 325 g de l'aminoalcool cherché, soit un rendement de 92,2%.

Exemple 4

Préparation du N(hydroxy-5′ oxa-3′ pentyl)aza-8 tetraoxa-3,6,11,14 pentadécane.

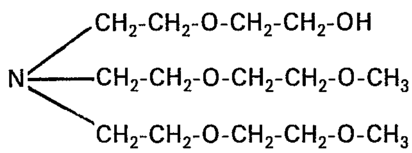

Dans les mêmes conditions qu'à l'exemple 1, on fait réagir:

- aza-8 tétraoxa-3,6,11,14 pentadécane 676,5 g (3 moles)
- diéthylèneglycol 1285 g (12 moles)
- nickel Raney déshydraté 218 g

On maintient 3 heures à 180—185°C sous un courant de 2g/h d'hydrogène.

Après séparation du nickel Raney, la masse est distillée pour récupérer l'excès de diéthylèneglycol et 662 g de l'aminoalcool attendu qui bout à $Eb_1$ = 200°C.

Le rendement par rapport à l'amine secondaire engagée est de 71,5%.

Exemple 5

Préparation du N(hydroxy-8′ dioxa-3′,6′ octyl)aza-8 tétraoxa-2,5,11,14 pentadécane.

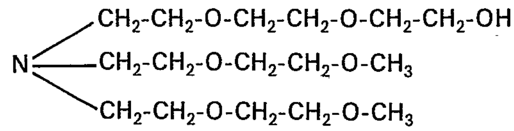

Dans les mêmes conditions qu'à l'exemple 1, on fait réagir:

- triéthylène glycol 890 g (6 moles)
- Ni Raney déshydraté 160 g
- aza-8 tétraoxa-2,5,11, 14 pentadécane 442 g (2 moles)

Après 3 heures de chauffage à 180°C, filtration du nickel Raney et distillation pour éliminer le triéthylène glycol, on récupère 508 g de l'aminoalcool attendu qui bout à $Eb_{0,3}$ = 193°C.

Le rendement par rapport à l'amine secondaire est de 72%.

Exemple 6

Propriété complexante du N(hydroxy 8′ dioxa 3′,6′ octyl)aza II hexaoxa 2,5,8,14,17,20 heneicosane.

Solubilisation des thiocyanates alcalins dans le chlorure de méthylène.

Ce test mettant en évidence la propriété complexante du produit préparé à l'exemple 3 est réalisé comme suit:

Dans un erlenmeyer de 50 ml équipé d'un réfrigérant ascendant et d'un agitateur magnétique, on introduit 10 ml de chlorure de méthylène anhydre et purifié (c'est-à-dire exempt de stabilisant).

On ajoute ensuite:

- 0,001 mole d'un thiocyanate alcalin choisi parmi les thiocyanates de Li, Na et K;
- 0,001 mole d'agent complexant à tester.

Après avoir agité le mélange pendant 10 mm à la température ambiante, on centrifuge; la solution claire ainsi obtenue est analysée par spectrométrie de flamme.

Le test est également réalisé en l'absence d'agent complexant à tester.

Les résultats obtenus sont les suivants:

- Solubilisation de Li S C N
- solubilité mesurée: 690 mg/l
- solubilité maximum calculée: 690 mg/l
- taux de solubilisation: 100%
- complexes formé:

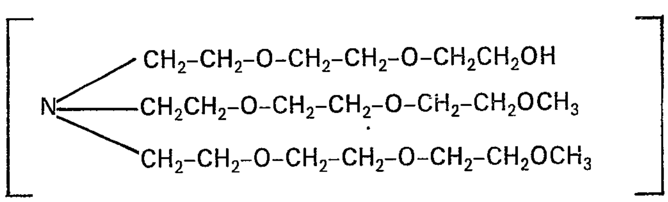
LiSCN

- Solubilité mesurée sans agent complexant: <1 mg/l

- Solubilisation de NaSCN
- Solubilité mesurée: 2300 mg/l

- solubilité maximum calculée: 2300 mg/l
- taux de solubilisation: 100%

- complexe formé:

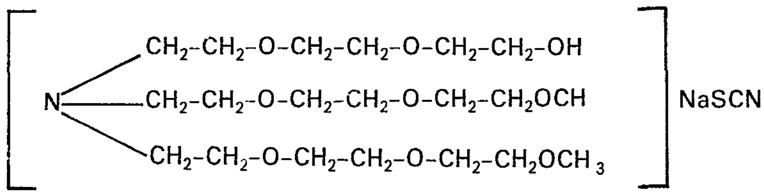

- Solubilité mesurée sans agent complexant: <1 mg/l

- Solubilisation de KSCN

- solubilité mesurée: 3380 mg/l
- solubilité maximum calculée: 3910 mg/l
- taux de solubilisation: 86,4%
- complexe formé:

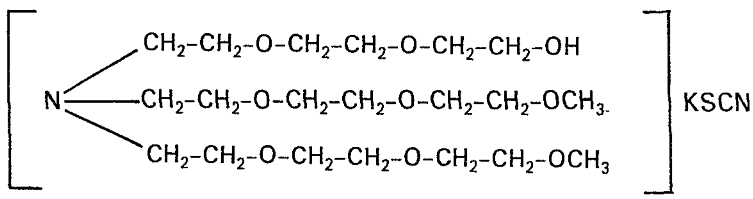

Les résultats ci-dessus peuvent être résumés dans le tableau suivant:

| sel à dissoudre | solubilité maximum calculée | solubilité mesurée en présence de l'agent | solubilité mesurée sans agent complexant |
|---|---|---|---|
| LiSCN | 690 mg/l | 690 mg/l | < 1 mg/l |
| NaSCN | 2300 mg/l | 2300 mg/l | < 1 mg/l |
| KSCN | 3910 mg/l | 3980 mg/l | < 1 mg/l |

Exemple 7

Propriété complexante du N(hydroxy-5′ oxa-3′ pentyl) aza-8 tétraoxa-3,6,11,14 pentadécane Solubilisation des thiocyantes alcalins dans le chlorure de méthylène.

Le test décrit à l'exemple 6 est réalisé en mettant en œuvre comme agent complexant à tester le produit de l'exemple 4.

Les résultats sont les suivants:

| sel à dissoudre | solubilité maximum calculée | solubilité mesurée en présence de l'agent | solubilité mesurée sans agent complexant |
|---|---|---|---|
| LiSCN | 690 mg/l | 670 mg/l | < 1 mg/l |
| NaSCN | 2300 mg/l | 2255 mg/l | < 1 mg/l |
| KSCN | 3910 mg/l | 3640 mg/l | < 1 mg/l |

- taux de solubilisation de LiSCN: 97%
  NaSCN: 98%
  KSCN: 93%

- complexes formés:

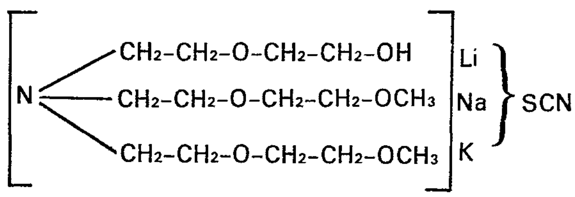

Exemple 8

Propriété complexante du N(hydroxy-8′ dioxa-3′,6′ octyl/aza-8 tétraoxa-2,5,11,14 pentadécane

Solubilisation des thiocyanates alcalins dans le chlorure de méthylène.

Le test décrit à l'exemple 6 est réalisé en mettant en œuvre comme agent complexant à tester le produit de l'exemple 5. Les résultats sont les suivants:

| sel à dissoudre | solubilité maximum calculée | solubilité mesurée en présence de l'agent | solubilité mesurée sans agent complexant |
|---|---|---|---|
| LiSCN | 690 mg/l | 685 mg/l | < 1 mg/l |
| NaSCN | 2300 mg/l | 2300 mg/l | < 1 mg/l |
| KSCN | 3910 mg/l | 3640 mg/l | < 1 mg/l |

- taux de solubilisation de LiSCN: 99%
  NaSCN: 100%
  KSCN: 93%

- complexes formés:

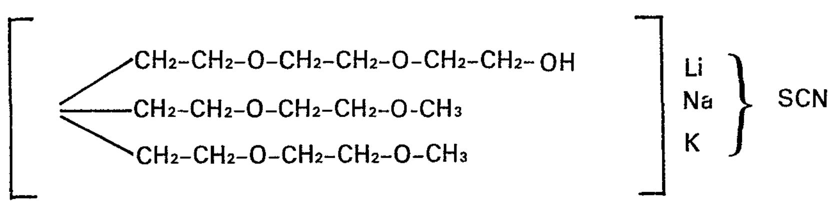

**Revendications**

1. Procédé de préparation d'aminoalcools polyoxaalkylés ou polyoxaarylés de formule:

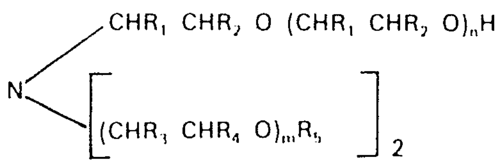

(I)

formule dans laquelle:

– $R_1$, $R_2$, $R_3$, $R_4$ sont semblables ou différents et représentent:

• un atome d'hydrogène

• un radical alkyle contenant de 1 à 4 atomes de carbone

– $R_5$ représente:

• un radical alkyle contenant de 1 à 12 atomes de carbone, éventuellement substitué par un groupe phényle,

• un radical cycloalkyle contenant de 3 à 12 atomes de carbone,

• un radical phényle éventuellement substitué par un groupe alkyle contenant de 1 à 12 atomes de carbone.

– n est un nombre entier supérieur ou égal à 1 et inférieur ou égal à 9

– m est un nombre entier supérieur ou égal à 1 et inférieur ou égal à 10

ledit procédé étant caractérisé en ce que l'on fait réagir un polyalkylèneglycol de formule:

$$HO\text{-}CHR_1\text{-}CHR_2\text{-}O\text{-}(CHR_1\text{-}CHR_2\text{-}O)_nH \qquad (II)$$

avec une bis(polyoxaalkyl)amine ou bis(polyoxaaryl)amine de formule:

$$HN\text{-}\left[(CHR_3\text{-}CHR_4\text{-}O)_mR_5\right]_2 \qquad (III)$$

selon un rapport molaire polyalkylène glycol de formule (II)/bis-(polyoxaalkyl)amine ou bis(polyoxaaryl)amine de formule III d'au moins 1,5 fois le rapport stoechiométriquement requis pour l'obtention d'un aminoalcool polyoxaalkyle ou polyoxaaryle de formule I, en présence d'un catalyseur d'hydrogénation-déshydrogénation, à une température comprise entre 120 et 220°C et en ce que l'on sépare l'aminoalcool polyoxaalkylé ou polyoxaarylé de formule I.

2. Procédé selon la revendication 1, caractérisé en ce que:

– $R_1$,$R_2$, $R_3$, $R_4$ sont semblables ou différents et représentent:

• un atome d'hydrogène

• un radical méthyle

– $R_5$ représente:

• un radical alkyle contenant de 1 à 6 atomes de carbone, éventuellement substitué par un groupe phényle;

• un radical cyclohexyle

• un radical phényle éventuellement substitué par un groupe alkyle contenant de 1 à 4 atomes de carbone

– n représente un nombre entier supérieur ou égal à 1 et inférieur ou égal à 5,

– m représente un nombre entier supérieur ou égal à 1 et inférieur ou égal à 6.

3. Procédé selon la revendication 1 ou la revendication 2, caractérisé en ce que lorsque $R_5$ représente un radical alkyle il contient de 1 à 4 atomes de carbone.

4. Procédé selon la revendication 1 ou la revendication 2, caractérisé en ce que n est égal à 1, 2 ou 3 et m égal à 1, 2, 3 ou 4.

5. Procédé selon l'une quelconque des revendications 1 à 4, caractérisé en ce que la réaction entre le polyalkylèneglycol et la bis(polyoxaaryl)amine est réalisée à une température comprise entre 150 et 200°C.

6. Procédé selon l'une quelconque des revendications 1 à 4, caractérisé en ce que le catalyseur est choisi parmi les catalyseurs nickel Raney et Harshaw, et est utilisé selon une quantité comprise entre 1 et 15% du poids de bis(polyoxaalkyl)amine ou bis(polyoxaaryl)amine.

7. Procédé selon la revendication 6, caractérisé en ce que la quantité de catalyseur est comprise entre 2 et 6% du poids de bis(polyoxaalkyl)amine ou bis(polyoxaaryl)amine.

8. Procédé selon l'une quelconque des revendications 1 à 4, caractérisé en ce que le rapport molaire polyalkylèneglykol/bis(polyoxaalkyl)amine ou bis(polyoxaaryl)amine est compris entre 1,5 et 10 fois la stoechiométrie.

9. Procédé selon la revendication 8, caractérisé en ce que ledit rapport molaire est compris entre 2 et 6 fois la stoechiométrie.

10. Procédé selon l'une quelconque des revendications 1 à 4, caractérisé en ce que la réaction entre le polyalkylèneglykol et la bis(polyoxaalkyl) amine ou bis(polyoxaaryl)amine est réalisée en présence d'hydrogène selon une quantité correspondant à 1–10% du poids d'alkylèneglykol.

11. Procédé selon la revendication 10, caractérisé en ce que la quantité d'hydrogène correspond à 1 à 5% du poids d'alkylèneglykol.

12. Procédé selon la revendication 1, caractérisé en ce que l'on fait réagir du di-, tri- ou tétraéthylèneglycol sur une bis(polyoxaalkyl)amine de formule:

$$HN\left[(CH_2\text{-}CH_2\text{-}O)_mC_pH_{2p+1}\right]_2$$

où m est égal à 1, 2 ou 3 et p égal à 1, 2, 3 ou 4 et en ce que l'on sépare l'aminoalcool polyoxyalkylé de formule:

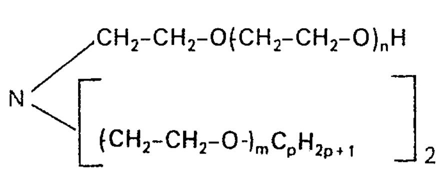

où n est égal respectivement à 1, 2 ou 3.

13. Procédé selon la revendication 1, caractérisé en ce que l'on fait réagir du di-, tri-, ou tétraéthylèneglycol sur une bis (polyoxaaryl) amine de formule:

$$HN\left[(CH_2-CH_2-O)_mC_6H_5\right]_2$$

où m est égal à 1, 2 ou 3 et en ce que l'on sépare l'aminoalcool polyoxarylé de formule:

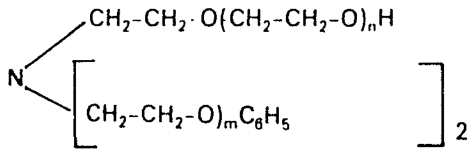

où n est égal respectivement à 1, 2 ou 3.

14. Application des aminoalcools polyoxaalkylés ou polyoxaarylés obtenu faisant selon l'une quelconque des revendications précédentes comme agents de complexation de cations.

**Patentansprüche**

1. Verfahren zur Herstellung von polyoxalkylierten oder polyoxarylierten Aminoalkoholen der Formel:

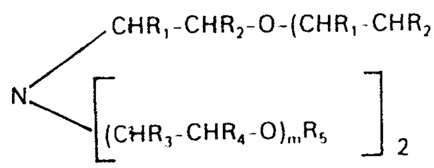

(I)

in der:

- $R_1$, $R_2$, $R_3$, $R_4$ gleich oder verschieden ein Wasserstoffatom oder ein Alkylradikal mit 1 bis 4 Kohlenstoffatomen bedeuten
- $R_5$ einen gegebenenfalls durch eine Phenylgruppe substituierten Alkylrest mit 1 bis 12 Kohlenstoffatomen, einen Cycloalkylrest mit 3 bis 12 Kohlenstoffatomen oder einen gegebenenfalls mit einer 1 bis 12 Kohlenstoffatome enthaltenden Alkylgruppe substituierten Phenylrest bedeuten,
- n eine ganze Zahl grösser gleich 1 und kleiner oder gleich 9 ist und
- m eine ganze Zahl grösser oder gleich 1 und kleiner oder gleich 10 ist,

wobei das Verfahren dadurch gekennzeichnet ist, dass man ein Polyäthylenglykol der Formel:

$$HO-CHR_1-CHR_2-O-(CHR_1-CHR_2-O)_nH \quad (II)$$

mit einem Bis(polyoxaalkyl)amin oder Bis(polyoxaaryl)-amin der Formel:

$$HN\left[(CHR_3-CHR_4-O)_mR_5\right]_2 \quad (III)$$

in einem Molverhältnis des Polyalkylenglykols der Formel II zum Bis(polyoxaalkyl)amin oder Bis(polyoxaaryl)amin der Formel III von wenigstens dem 1,5-fachen der stöchiometrisch für die Herstellung des polyoxalkylierten oder polyoxarylierten Aminoalkohols der Formel I erforderlichen Mengen in Gegenwart eines Hydrierungs-Dehydrierungskatalysators bei einer Temperatur zwischen 120 und 220°C umsetzt und den polyoxalkylierten oder polyoxarylierten Aminoalkohol der Formel I gewinnt.

2. Verfahren nach Anspruch 1, dadurch gekennzeichnet, dass

- $R_1$, $R_2$, $R_3$, $R_4$ gleich oder verschieden ein Wasserstoffatom oder einen Methylrest,
- $R_5$ einen gegebenenfalls durch eine Phenylgruppe substituierten Alkylrest mit 1 bis 6 Kohlenstoffatomen, einen Cyclohexylrest oder einen gegebenenfalls durch eine Alkylgruppe mit 1 bis 4 Kohlenstoffatomen substituierten Phenylrest bedeutet,
- n eine ganze Zahl grösser oder gleich 1 und kleiner oder gleich 5 und
- m eine ganze Zahl grösser oder kleiner 1 und kleiner oder gleich 6 ist.

3. Verfahren nach Anspruch 1 oder 2, dadurch gekennzeichnet, dass dann wenn $R_5$ einen Alkylrest bedeutet dieser 1 bis 4 Kohlenstoffatome enthält.

4. Verfahren nach Anspruch 1 oder 2, dadurch gekennzeichnet, dass n gleich 1, 2 oder 3 und m gleich 1, 2, 3 oder 4 ist.

5. Verfahren nach irgendeinem der Ansprüche 1 bis 4, dadurch gekennzeichnet, dass die Reaktion zwischen dem Polyalkylenglykol und dem Bis(polyoxaalkyl)amin oder Bis(polyoxaaryl)amin bei einer Temperatur zwischen 150 und 200°C durchgeführt wird.

6. Verfahren nach irgendeinem der Ansprüche 1 bis 4, dadurch gekennzeichnet, dass der Katalysator unter den Raney- und Harshaw-Nickelkatalysatoren ausgewählt ist und in einer Menge von 1 bis 15 Gewichtsprozent des Bis(polyoxaalkyl)amins oder Bis(polyoxaaryl)amins angewendet wird.

7. Verfahren nach Anspruch 6, dadurch gekennzeichnet, dass die Menge des Katalysators 2 bis 6% des Gewichts des Bis(polyoxaalkyl)amins oder Bis(polyoxaaryl)amins beträgt.

8. Verfahren nach irgendeinem der Ansprüche 1 bis 4, dadurch gekennzeichnet, dass das Molverhältnis Polyalkylenglykol zu Bis(polyoxaalkyl)amin oder Bis(polyoxaaryl)amin das 1,5 bis 10fache der Stöchiometrie beträgt.

9. Verfahren nach Anspruch 8, dadurch gekennzeichnet, dass das genannte Molverhältnis das 2 bis 6fache der Stöchiometrie beträgt.

10. Verfahren nach irgendeinem der Ansprüche 1 bis 4, dadurch gekennzeichnet, dass die Reaktion zwischen dem Polyalkylenglykol und dem Bis(polyoxaalkyl)amin oder Bis(polyxaaryl)amin in Gegenwart von Wasserstoff entsprechend einer Menge von 1 bis 10% des Gewichts des Alkylenglykols durchgeführt wird.

11. Verfahren nach Anspruch 10, dadurch gekennzeichnet, dass die Menge des Wasserstoffs 1 bis 5% des Gewichtes des Alkylenglykols entspricht.

12. Verfahren nach Anspruch 1, dadurch gekennzeichnet, dass man Di-, Tri- oder Tetra-äthylenglykol auf ein Bis(polyoxaalkyl)amin der Formel:

$$HN\left[(CH_2-CH_2-O)_mC_pH_{2p+1}\right]_2$$

einwirken lässt, in der m 1, 2 oder 3 und p 1, 2, 3 oder 4 ist und dass man den polyoxalkylierten Aminoalkohol der Formel:

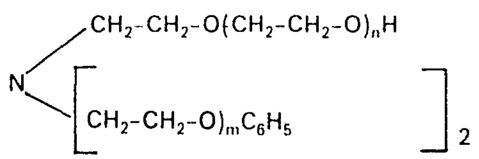

in der n 1, 2 oder 3 ist abtrennt.

13. Verfahren nach Anspruch 1, dadurch gekennzeichnet, dass man das Di-, Tri- oder Tetraäthylenglykol auf ein Bis(polyoxaaryl)amin der Formel:

$$HN\left[(CH_2\ \ CH_2-O)_mC_6H_5\right]_2$$

in der m gleich 1, 2 oder 3 ist einwirken lässt und dass man den erhaltenen polyoxarylierten Aminialkohol der Formel:

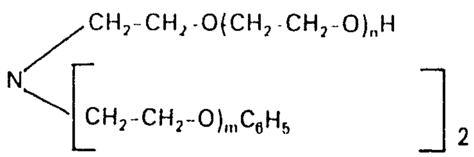

in der n 1, 2 oder 3 ist abtrennt.

14. Verwendung der nach irgendeinem der vorangehenden Patentansprüche erhaltenen polyoxalkylierten oder polyoxarylierten Aminoalkohole als Komplexbildungsmittel für Kationen.

## Claims

1. Process for the preparation of polyoxaalkylated or polyoxaarylated aminoalcohols of the formula:

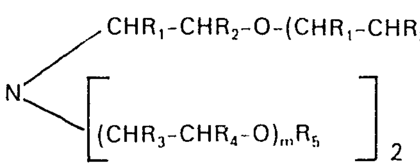

(I)

in which formula:

– $R_1$, $R_2$, $R_3$ and $R_4$ are similar or different and represent:

· a hydrogen atom or

· an alkyl radical containing from 1 to 4 carbon atoms,

– $R_5$ represents:

· an alkyl radical containing from 1 to 12 carbon atoms, which is optionally substituted by a phenyl group,

· a cycloalkyl radical containing from 3 to 12 carbon atoms, or

· a phenyl radical optionally substituted by an alkyl group containing from 1 to 12 carbon atoms,

– n is an integrer greater than or equal to 1 and less than or equal to 9, and

– m is an integrer greater than or equal to 1 and less than or equal to 10, the said process being characterised in that a polyalkylene glycol of the formula:

(II) $HO-CHR_1-CHR_2-O-(CHR_1-CHR_2-O)_nH$

is reacted with a bis-(polyoxaalkyl)-amine or bis-(polyoxaaryl)-amine of the formula:

$$HN\left[(CHR_3-CHR_4-O)_mR_5\right]_2 \quad (III)$$

in a molar ratio of polyalkylene glycol of the formula II/bis-(polyoxaalkyl)-amine or bis-(polyoxaaryl)-amine of the formula III which is at least 1.5 times the ratio stoichiometrically required to obtain a polyoxaalkylated or polyoxaarylated aminoalcohol of the formula I, in the presence of a hydrogenation/dehydrogenation catalyst, at a temperature of between 120 and 220°C, and in that the polyoxaalkylated or polyoxaarylated aminoalcohol of the formula I is separated off.

2. Process according to Claim 1, characterised in that:

– $R_1$, $R_2$, $R_3$ and $R_4$ are similar or different and represent:

· a hydrogen atom or

· a methyl radical,

– $R_5$ represents:

· an alkyl radical containing from 1 to 6 carbon atoms, which is optionally substituted by a phenyl group,

· a cyclohexyl radical or

· a phenyl radical optionally substituted by an alkyl group containing from 1 to 4 carbon atoms,

– n represents an integrer greater than or equal to 1 and less than or equal to 5, and

– m represents an integer greater than or equal to 1 and less than or equal to 6.

3. Process according to Claim 1 or Claim 2, characterised in that if $R_5$ represents an alkyl radical, it contains from 1 to 4 carbon atoms.

4. Process according to Claim 1 or Claim 2, characterised in that n is equal to 1, 2 or 3 and m is equal to 1, 2, 3 or 4.

5. Process according to any one of Claims 1 to 4, characterised in that the reaction of the polyalkyleneglycol with the bis-(polyoxaalkyl)-amine or bis-(polyoxaaryl)-amine is carried out at a temperature of between 150 and 200°C.

6. Process according to any one of Claims 1 to

4, characterised in that the catalyst is chosen from amongst Raney and Harshaw nickel catalysts and is used in an amount of between 1 and 15% of the weight of bis(polyoxaalkyl)-amine or bis-(polyoxaaryl)-amine.

7. Process according to Claim 6, characterised in that the amount of catalyst is between 2 and 6% of the weight of bis-(polyoxaalkyl)-amine or bis-(polyoxaaryl)-amine.

8. Process according to any one of Claims 1 to 4, characterised in that the molar ratio of polyalkylene glycol/bis-(polyoxaalkyl)-amine or bis-(polyoxaaryl)-amine is between 1.5 and 10 times the stoichiometric ratio.

9. Process according to Claim 8, characterised in that the said molar ratio is between 2 and 6 times the stoichiometric ratio.

10. Process according to any one of Claims 1 to 4, characterised in that the reaction of the polyalkylene glycol with the bis-(polyoxaalkyl)-amine or bis-(polyoxaaryl)-amine is carried out in the presence of hydrogen in an amount corresponding to 1-10% of the weight of alkylene glycol.

11. Process according to Claim 10, characterised in that the amount of hydrogen corresponds to 1 to 5% of the weight of alkylene glycol.

12. Process according to Claim 1, characterised in that di-, tri- or tetra-ethylene glycol is reacted with a bis-(polyoxaalkyl)-amine of the formula:

$$HN\left[(CH_2-CH_2-O)_mC_pH_{2p+1}\right]_2$$

in which m is equal to 1, 2 or 3 and p is equal to 1, 2, 3 or 4, and in that the polyoxyalkylated aminoalcohol of the formula:

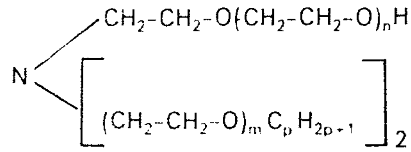

in which n is respectively equal to 1, 2 or 3, is separated off.

13. Process according to Claim 1, characterised in that di-, tri- or tetra-ethylene glycol is reacted with a bis-(polyoxaaryl)-amine of the formula:

$$HN\left[(CH_2-CH_2-O)_mC_6H_5\right]_2$$

in which m is equal to 1, 2 or 3, and in that the polyoxyarylated aminoalcohol of the formula:

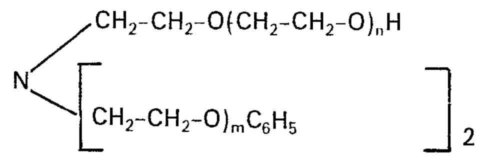

in which n is respectively equal to 1, 2 or 3, is separated off.

14. Application of the polyoxaalkylated or polyoxaarylated aminoalcohols obtained according to any one of the preceding claims, as complexing agents for cations.